(19) Europäisches Patentamt
European Patent Office
Office européen des brevets

(11) Veröffentlichungsnummer: **0 002 757**
**B1**

(12) **EUROPÄISCHE PATENTSCHRIFT**

(45) Veröffentlichungstag der Patentschrift: **25.11.81**

(21) Anmeldenummer: **78101726.4**

(22) Anmeldetag: **16.12.78**

(51) Int. Cl.³: **C 07 C 149/20,** C 07 C 153/09, A 01 N 37/38, A 01 N 43/00, A 01 N 37/18

# (54) Phenoxy-phenylthio-milchsäurederivate, deren Herstellung, sie enthaltende herbizide und pflanzenwuchsregulierende Mittel sowie deren Verwendung.

(30) Priorität: **28.12.77 CH 16107/77**

(43) Veröffentlichungstag der Anmeldung:
**11.07.79 Patentblatt 79/14**

(45) Bekanntmachung des Hinweises auf die Patenterteilung:
**25.11.81 Patentblatt 81/47**

(84) Benannte Vertragsstaaten:
**BE CH DE FR GB IT NL**

(56) Entgegenhaltungen:

FR - A - 2 325 638

CHEMICAL ABSTRACTS, Vol. 88, Nr. 21, 22. Mai 1978, Zusammenfassung Nr. 152221c, Columbus, Ohio, USA, YOSHIMOTO TAKEO et al., "Diphenylethers", Seite 570, rechte Spalte

CHEMICAL ABSTRACTS, Vol. 87, Nr. 15, 10. Oktober 1977, Zusammenfassung Nr. 117670f Columbus, Ohio, USA, YOSHIMOTO TAKEO et al., "Herbicidal diphenyl ethers", Seite 579, linke Spalte

(73) Patentinhaber: **CIBA-GEIGY AG**
**Patentabteilung Postfach**
**CH-4002 Basel (CH)**

(72) Erfinder: **Dürr, Dieter, Dr.**
**Brändelistalweg 16**
**CH-4103 Bottmingen (CH)**
Erfinder: **Rohr, Otto, Dr.**
**Kilbertweg 19**
**CH-4106 Therwil (CH)**
Erfinder: **Pissiotas, Georg, Dr.**
**Breslauerstrasse 8**
**D-7850 Lörrach (DE)**
Erfinder: **Böhner, Beat, Dr.**
**Hügelweg 3**
**CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 757 B1

Courier Press, Leamington Spa, England.

# Phenoxy-phenylthio-milchsäurederivate, deren Herstellung, sie enthaltende herbizide und pflanzenwuchsregulierende Mittel sowie deren Verwendung

Die vorliegende Erfindung betrifft neue Phenoxy-phenylthio-milchsäurederivate, welche herbizide und das Pflanzenwashstum regulierende Wirkung zeigen, Verfahren zur Herstellung dieser Derivate, herbizide und das Pflanzenwachstum regulierendes Mittel welche diese Derivate als Wirkstoffe enthalten sowie die Verwendung der neuen Phenoxy-phenylthio-milchsäurederivate oder der sie enthaltenden Mittel als Herbizide und/oder zur Regulierung des Pflanzenwachstums.

Die neuen Phenoxy-phenylthio-milchsäurederivate entsprechen der Formel I

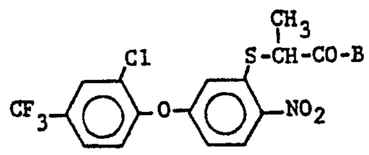

(I)

In dieser Formel bedeuten

B einen Rest —OR, —SR oder —$NR_1R_2$,

R Wasserstoff, das Kation eines Alkali- oder Erdalkalimetalles oder einer Ammoniumgruppe, einen $C_1$—$C_{18}$ *Alkylrest,* der unsubstituiert oder ein- oder mehrfach durch Halogen, $C_1$—$C_4$ Alkoxy, $C_1$—$C_4$ Alkylthio, $C_3$—$C_8$ Cycloalkyl, Nitro oder Cyano substituiert sein kann, $C_3$—$C_{18}$ *Alkenyl,* unsubstituiert oder durch Halogen substituiert, $C_3$—$C_{18}$ *Alkinyl, Phenyl* oder *Benzyl* unsubstituiert oder substituiert durch Halogen, $C_1$—$C_4$ Alkyl, $C_1$—$C_4$ Alkoxy, $C_1$—$C_4$ Alkylthio, Trifluormethyl, Nitro oder Cyano,

$R_1$ *Wasserstoff, $C_1$—$C_4$ Alkyl,* gegebenenfalls substituiert durch Halogen, Cyano, Hydroxyl oder $C_1$—$C_4$ Alkoxy, $C_3$—$C_6$ *Alkenyl,* $C_3$—$C_6$ *Alkinyl,* $C_1$—$C_4$ *Alkoxy, Phenyl* oder *Benzyl,* gegebenenfalls substituiert durch Halogen, Trifluormethyl, Nitro, Cyano, $C_1$—$C_4$ Alkyl, $C_1$—$C_4$ Alkoxy, $C_1$—$C_4$ Alkylthio,

$R_2$ *Wasserstoff,* $C_1$—$C_4$ *Alkyl* oder $C_3$—$C_6$ *Alkenyl,*

$R_1$ und $R_2$ zusammen mit dem Stickstoffatom an das sie gebunden sind auch einen 5—7 gliedrigen *Heterocyclus* der auch Sauerstoff, Schwefel oder eine Gruppe >$NR_2$ im Ring enthalten kann.

Die Alkylreste in dieser Formel können verzweigt oder unverzweigt sein und enthalten die angegebene Anzahl Kohlenstoffatome Ammoniumgruppen können neben Wasserstoff noch $C_1$—$C_4$ Alkyl oder Hydroxyalkylreste enthalten.

Die neuen Wirkstoffe der Formel I gemäss vorliegender Erfindung besitzen Herbizidwirkung bei pre- und post-emergenter Anwendung und können in mono- und dikotylen Kulturen als Unkrautmittel eingesetzt werden.

Ferner besitzen sie günstige wachstumsregulierende Effekte (Wuchshemmung). Insbesondere hemmen sie das Wachstum von dicotylen Pflanzen. Beispiele für die nutzbringende Anwendung der erfindungsgemässen Verbindungen sind z.B.

— die Reduktion des vegetativen Wachstums bei Soja und ähnlichen Leguminosen, was zu einer Ertragssteigerung dieser Kultur führt;
— die Hemmung des unerwünschten Wachstums von Geiztrieben bei Tabak, dessen Haupttrieb man geschnitten hat, was der Ausbildung grössere und schönere Blätter zugute kommt;
— die Hemmung des Wachstums von Gras und dikotyledonen Pflanzen, wie Obstbäume, Zierbäume, Gebüsche und Hecken, zwecks Einsparung an Schnittarbeit.

Die Verbindungen vorliegender Erfindung sind wenig giftig für Warmblüter und deren Applikation wirft keine Probleme auf. Die Aufwandmenge liegt zwischen 0,1 und 5 kg pro Hektar.

Die Herstellung der neuen Verbindungen der Formel I erfolgt nach an sich bekannten Methoden und kann aus dem folgendem Formelschema ersehen werden.

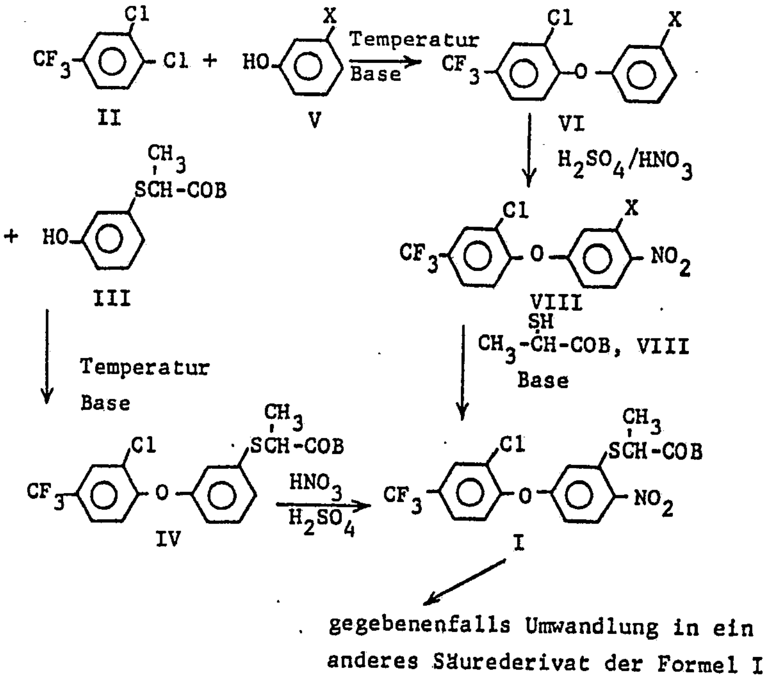


In diesen Formeln hat B die unter Formel I gegebene Bedeutung, X ist ein Halogenatom oder die Nitrogruppe.

Man kondensiert beispielsweise 1,2-Dichlor-4-trifluorbenzol der Formel II mit einem in meta-Stellung substituierten Phenol der Formel III oder V, wobei man in relativ guter Ausbeute einen Diphenyläther der Formel IV oder VI erhält, welcher dann bei tiefer Temperatur die zwischen —10°C und Raumtemperatur liegt mittels einem Salpeter- und Schwefelsäuregemisch nitriert.

Die Kondensationen werden vorteilhafterweise bei erhöhter Temperatur (Raumtemperatur bis zum Siedepunkt des Reaktionsgemisches) in einem den Reaktionspartnern gegenüber inerten, organischen Lösungsmittel vorgenommen, in Anwesenheit der äquimolaren Menge einer anorganischen oder organischen Base als säurebindendes Mittel.

Ein erfinderisches Verfahren zur Herstellung der Phenoxy-phenylthio-milchsäurederivater der Formel I ist dadurch gekennzeichnet, dass man ein Phenoxy-phenylthio-milchsäurederivat der Formel IV

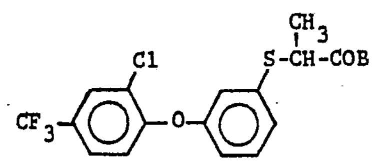

IV

worin B die unter Formel I gegebene Bedeutung hat, bei einer Temperatur, zwischen —10°C und Raumtemperatur mit einem Gemisch aus Salpeter- und Schwefelsäure nitriert.

Ein anderes erfinderisches Verfahren besteht darin, dass man einen in der 3'-Stellung durch Halogen oder Nitro substituierten 2-Chlor-4-trifluormethyl-(4'-nitro)-diphenyläther der Formel VII,

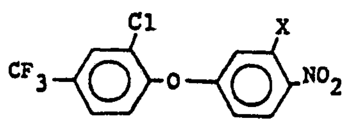

VII

worin X Halogen oder die Nitrogruppe bedeutet, in einem inerten organischen Lösungsmittel in Anwesenheit der äquimolaren Menge einer anorganischen oder organischen Base mit einem Derivat der 2-Thiomilchsäure der Formel VIII,

SH
|
$CH_3$—CH—COB     VIII

worin B die unter Formel I gegebene Bedeutung hat, kondensiert.

Einzelne Ausgangsverbindungen der Formeln IV und VI, sowie deren Herstellung sind bekannt und beschrieben z.B. in den DOS 2 130 919 oder 2 333 848.

Den Phenoxy-phenylthio-milchsäurederivate der Formel I ähnliche, aber vom Gegenstand dieser Erfindung nicht umfasste Verbindungen sind in den offengelegten Japanischen Patentanmeldungen No. 74—146 792, 75—147 443, 77—142 034 sowie 77—21 320 beschrieben worden.

Aehnlich konstituierte Phenoxy-$\alpha$-phenyl-milchsäurederivate sind z.B. ebenfalls aus der französischen Patentschrift No. 2,325,638 bekannt geworden. All diese Verbindungen weisen gute herbizide Eigenschaften auf, speziell gegen zweikeimblättrige Unkräuter beim Einsatz im Nachauflaufverfahren. Sie eignen sich, soweit sie nicht zu phytotoxisch sind, als selektive Herbizide in Reiskulturen und gewissen Getreidekulturen. Ueberraschenderweise hat sich gezeigt, dass die erfindungsgemässen Verbindungen sich im Gegensatz zu den bekannten auch gegenüber Kulturhirse (sorghum hybridum) selektiv verhalten und daher auch zur Bekämpfung von Unkräutern in dieser Kultur in Frage kommen.

Das nachfolgende Beispiel veranschaulicht das erfindungsgemässe Verfahren für einen Wirkstoff der Formel I. Weitere in entsprechender Weise hergestellte Wirkstoffe sind in den anschliessenden Tabellen aufgeführt. Temperaturangaben beziehen sich jeweils auf Celsius-Grade. Die Drücke sind in millibar angegeben.

Die Phenoxy-phenylthio-milchsäurederivate der Formel I sind stabile Verbindungen, welche in üblichen organischen Lösungsmitteln, wie Alkoholen, Aether, Ketonen, Dimethylformamid, Dimethylsulfoxyd etc. löslich sind.

Beispiel

$\alpha$ - [3 - (2' - Chlor - 4' - trifluoromethylphenoxy) - 6 - nitrophenylthio] - milchsäure - methylester

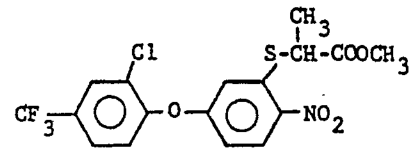

50 g 3-Nitro-2'-chlor-4'-trifluormethyl-diphenyläther wurden in 200 ml konz. Schwefelsäure und 100 ml Aethylenchlorid vorgelegt. Unter gutem Rühren trägt man in kleinen Portionen insgesamt 16,5 g Kaliumnitrat bei 0° ein. dann lässt man einige Zeit bei Raumtemperatur nachrühren. Zur Aufarbeitung giesst man das Reaktions-gemisch auf Eis, trennt die organische Schicht ab, wäscht mit Wasser neutral, dampft ein und reinigt den Rückstand durch Destillation oder Kristallisation. Man erhält so in guter Ausbeute 3,4-Dinitro-2'-chlor-4'-trifluormethyl-diphenyläther. Siedepunkt 180°/0,04 mm Hg (Kugelrohrofen), Smp. 75—81°.

20 g dieser Verbindung wurden in 70 ml Dimethylsulfoxyd mit 6,8 g Thiomilchsäuremethyläther und 2,3 g Natriumhydroxyd bei 10° umrührt. Nach 4 Stunden erhitzt man zur Vervollständigung der Reaktion auf 60°, giesst die warme Reaktionsmasse auf Eis und extrahiert mit Chloroform. Der Chloroformextrakt wird mit Wasser gewaschen, getrocknet, eingedampft und der Rückstand im Kugelrohrofen destilliert. Man erhält 15 g der Titelverbindung vom Sdp. 190—195°/0,04 millibar als rohes Oel. (Verbindung No. 1).

In analoger Weise erhält man bei singemässer Anwendung entsprechender Ausgangsprodukte folgende Verbindungen der Formel I

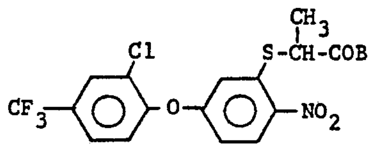

| Verbindung No. | B | physikalische Konstante |
|---|---|---|
| 1 | $OCH_3$ | Sdp. 190–95°/0.04 millibar |
| 2 | $OC_2H_5$ | Oel |
| 3 | $SCH_3$ | Wachs |
| 4 | $N(CH_3)_2$ | Oel |
| 5 | $N(CH_3)OCH_3$ | Oel |
| 6 | $OC_4H_9$ | Oel |
| 7 | $SCH_2{-}CH{=}CH_2$ | Oel |
| 8 | $OC_2H_4OC_2H_5$ | Sdp. 250°/0.03 millibar |
| 9 | $S{-}CH_2$ 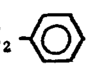 | Oel |
| 10 | OH | Oel |
| 11 | $OCH_2CN$ | Oel |
| 12 | $OC_2H_4CN$ | Oel |
| 13 | $OC_3H_6CN$ | Oel |
| 14 | $OC_2H_4Cl$ | Oel |
| 15 | $OC_3H_6Cl$ | Oel |
| 16 | $OC_{18}H_{37}$ | Oel |
| 17 | $OC_8H_{17}$ | Oel |
| 18 | O 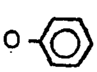 | |
| 19 | O 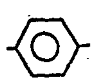 Cl | Oel |
| 20 | $OCH_2$ 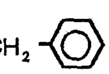 | Oel |
| 21 | $OCH_2CH{=}CH_2$ | Oel |
| 22 | $OCH_2C{\equiv}CH$ | Oel |
| 23 | $OCH_2{-}C{=}CH_2$ | Oel |

| Verbindung No. | B | physikalische Konstante |
|---|---|---|
| 24 | $O^{\ominus}$ $NH_4^{\oplus}$ | Wachs |
| 25 | $O^{\ominus}$ $NH_2^{\oplus}(C_2H_4OH)_2$ | Wachs |

Die Erfindung betrifft auch herbizide und pflanzenwashstumsregulierende Mittel, welche einen neuen Wirkstoff der Formel I enthalten, sowie Verfahren zur pre- und post-emergenten Unkrautbekämpfung und zur Hemmung des Pflanzenwuchses von monocotylen und dicotylen Pflanzen, insbesondere Gräsern, Getreide, Soja und Tabakgeiztrieben.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsgranulate Imprägnierungsgranulate und Homogengranulate; |
| in Wasser dispergierbare Wirkstoffkonzentrate: | Spritzpulver, (wettable powder), Pasten, Emulsionen: |
| flüssige Aufarbeitungsformen: | Lösungen. |

Der Gehalt an Wirkstoff in den oben beschriebenen Mitteln liegt zwischen 0,1 bis 95%, bevorzugt zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Die Wirkstoffe der Formel I können beispielsweise wie folgt formuliert werden (Teile bedeuten Gewichtsteile):

Die folgenden Beispiele für Aufbereitungsformen sollen die Herstellung der erfindungsgemässen Mittel näher erläutern.

*Stäubemittel:*
Zur Herstellung eines a) 5%igen und b) 2%igen Stäubemittels werden die folgenden Stoffe verwendet:

a) 5 Teile α - [3 - (2' - Chlor - 4' - trifluormethylphenoxy) - 6 - nitrophenylthio] - propionsäure - methylester,

95 Teile Talkum

b) 2 Teile des obigen Wirkstoffes

1 Teil hochdisperse Kieselsäure,

97 Teile Talkum;

Die Wirkstoffe werden mit den Trägerstoffen vermischt und vermahlen.

*Granulate*
Zur Herstellung eines 5%igen Granulates werden die folgenden Stoffe verwendet:

5 Teile des obigen Wirkstoffes

0,25 Teile Epichlorhydrin

0,25 Teile Cetylpoläthylenglykoläther mit 8 Mol Aethylenoxid,

3,50 Teile Polyäthylenglykol,

91 Teile Kaolin (Korngrösse 0,3 bis 0,8 mm)

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

*Spritzpulver*
Zur Herstellung eines a) 50%igen, b) 25%igen und c) 10%igen Spritzpulvers werden folgende Bestandteile verwendet:

| | | | |
|---|---|---|---|
| a) | 50 | Teile | $\alpha$ - [3 - (2' - Chlor - 4' - trifluormethylphenoxy) - 6 - nitrophenylthio] - propionsäure - methylester, |
| | 5 | Teile | Natriumdibutylnaphthylsulfonat, |
| | 3 | Teile | Naphthalinsulfonsäuren - Phenonolsulfonsäuren - Formaldheyd - Kondensat 3:2:1, |
| | 20 | Teile | Kaolin, |
| | 22 | Teile | Champagne-Kreide; |
| b) | 25 | Teile | des obigen Wirkstoffes, |
| | 5 | Teile | Oleylmethyltaurid-Natrium-Salz, |
| | 2,5 | Teile | Naphthalinsulfonsäuren-Formaldehyd-Kondensat, |
| | 0,5 | Teile | Carboxymethylcellulose, |
| | 5 | Teile | neutrales Kalium-Aluminium-Silikat, |
| | 62 | Teile | Kaolin; |
| c) | 10 | Teile | des obigen Wirkstoffs, |
| | 3 | Teile | Gemisch der Natriumsalze von gesättigten Fettalkoholen, |
| | 5 | Teile | Naphthalinsulfonsäuren-Formaldehyd-Kondensat, |
| | 82 | Teile | Kaolin. |

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen im Vorauflaufverfahren und zur Behandlung von Rasenanlagen verwendet.

*Paste*
Zur Herstellung einer 45%igen Paste werden folgende Stoffe verwendet:

| | | |
|---|---|---|
| 45 | Teile | $\alpha$ - [3 - (2' - Chlor - 4' - trifluormethylphenoxy) - 6 - nitrophenylthio] - propionsäure - methylester, |
| 5 | Teile | Natriumaluminiumsilikat, |
| 14 | Teile | Cetylpolyäthylenglykoläther mit 8 Mol Aethylenoxid, |
| 1 | Teil | Oleylpolyäthylenglykoläther mit 5 Mol Aethylenoxid, |
| 2 | Teile | Spindelöl, |
| 23 | Teile | Wasser, |
| 10 | Teile | Polyäthylenglykol. |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen. Die Suspensionen eignen sich zur Behandlung von Rasenanlagen.

*Emulsionskonzentrat*
Zur Herstellung eines 25%igen Emulsionskonzentrates werden

| | |
|---|---|
| 25 Teile | $\alpha$ - [3 - (2' - Chlor - 4' - trifluormethylphenoxy) - 6 - nitrophenylthio] - propionsäure - methylester, |
| 5 Teile | Mischung von Nonylphenolpolyoxyäthylen und Calcium-dodecylbenzol-sulfonat, |
| 35 Teile | 3,5,5-Trimethyl-2-cyclohexen-1-on, |
| 35 Teile | Dimethylformamid |

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere der von der Formel I umfassten Verbindungen verwendet werden.

Die in den erfindungsgemässen Mitteln enthaltenen Wirkstoffe beeinflussen das Pflanzenwachstum in verschiedener Weise. So hemmen, verzögern oder unterbinden sie in erster Linie das Wachstum und die Keimung. Es handelt sich dabei also sowohl um pre- und post-emergente Herbizidwirkung als auch um Wuchshemmung.

Erfindungsgemässe Mittel, die als aktive Komponente mindestens eine Verbindung der Formel I enthalten, eignen sich besonders zur Hemmung und Kontrolle des Pflanzenwachstums von monocotylen und dicotylen Pflanzen, wie Gräsern, Sträuchern, Bäumen, Getreide- und Leguminosenkulturen, Zuckerrohr, Tabak, Soja, Zwiebeln- und Kartoffelknollen, Zierpflanzen, Obstbäumen und Reben.

Die von den neuen Wirkstoffen der Formel I in erster Linie erzielte Wirkung besteht in der gewünschten Reduktion der Pflanzengrösse, insbesondere der Wuchshöhe. Im allgemeinen ist damit eine gewisse Aenderung der Pflanzenform verbunden. In unmittelbarem Zusammenhang zur Verminderung der Wuchshöhe erfährt die Pflanze eine Festigung. Blätter und Stengel sind kräftiger ausgebildet. Durch Verkürzung der Internodienabstände an monocotylen Pflanzen wird die Knickfestigkeit erhöht. Auf diese Weise können Ernteausfälle durch Gewittersturm, Dauerregen, usw., die normalerweise zu einem Lagern von Getreide- und Leguminosenkulturen führen, weitgehend verhindert und damit die Erntearbeit erleichtert werden. Als Nebeneffekt führt verminderte Wuchshöhe bei Nutzpflanzen zu einer Einsparung an Düngemitteln. In gleichem Masse gilt dies auch für Zierpflanzen, Zierrasen, Sportrasen oder sonstige Grünanpflanzungen.

Eines der wichtigsten Probleme an reinen Grasbepflanzungen ist jedoch der Grasschnitt selbst, sei es an Grünanlagen in Wohngegenden, auf Industriegeländen, auf Sportplätzen, an Autostrassen, Flugpisten, Eisenbahndämmen oder Uferböschungen von Gewässern. In all diesen Fällen ist ein periodisches Schneiden des Rasens bzw. des Graswurchses notwendig. Dies ist nicht nur im Hinblick auf Arbeitskräfte und Maschinen sehr aufwendig, sondern bringt im Verkehrsbereich auch erhebliche Gefahren für das betroffene Personal und die Verkehrsteilnehmer mit sich.

Es besteht daher gerade in Gebieten mit grossen Verkehrsnetzen ein dringendes Bedürfnis, die im Hinblick auf die Verfestigung von Seitenstreifen und Böschungen an Verkehrsvegen notwendige Grasnarbe einerseits zu erhalten und zu pflegen, andererseits aber mit einfachen Massnahmen während der gesamten Vegetationsperiode auf einer mittleren Wuchshöhe zu halten. Dies wird durch Applikation erfindungsgemässer Wirkstoffe der Formel I auf sehr günstige Weise erreicht.

In analoger Weise kann durch Behandlung von Bäumen, Sträuchern und Hecken, vor allem in Wohnund Industriegebieten, mit erfindungsgemässen Verbindungen der Formel I die arbeitsaufwendige Schnittarbeit reduziert werden.

Durch den Einsatz erfindungsgemässer Wirkstoffe der Formel I können auch das Triebwachstum und/oder die Fruchtbarkeit von Obstbäumen und Reben vorteilhaft beeinflusst werden.

Zierpflanzen mit starkem Längenwachstum können durch Behandlung mit erfindungsgemässen Wirkstoffen als kompakte Topfpflanzen gezogen werden.

Die Wirkstoffe der Formel I finden auch Anwendung zur Hemmung des Wachstums unerwünschter Geiztriebe, z.B. bei Tabak und Zierpflanzen, wodurch das arbeitsintensive Ausbrechen dieser Triebe von Hand vermieden wird, ferner zur Austriebhemmung bei lagernden Knollen, beispielsweise bei Zierpflanzenknollen, bei Zwiebeln und Kartoffeln, und schliesslich zur Ertragssteigerung bei stark vegetativ wachsenden Kulturpflanzen, wie Soja und Zuckerrohr, indem durch Applikation erfindungsgemässer Wirkstoffe der Uebergang von der vegetativen zur generativen Wachstumsphase beschleunigt wird.

Bevorzugt setzt man die erfindungsgemässen Wirkstoffe der Formel I zur Wachstumshemmung an Gräsern, Getreidekulturen, Tabak, Soja und Zierpflanzen ein.

Die Aufwandmengen sind verschieden und vom Applikationszeitpunkt abhängig. Sie liegen im allgemeinen zwischen 0,1 und 5 kg Wirkstoff pro Hektar, bei Applikation vor dem Auflaufen der Pflanzen und für die Behandlung von bestehenden Kulturen vorzugsweise bis zu 4 kg pro Hektar.

Ferner eignen sich viele der Wirkstoffe der Formel I und diese enthaltende Mittel auch für andere Beeinflussungen des Pflanzenwachstums, wie insbesondere zur Erleichterung der Frucht- und Blattabszission durch Ausbildung von Trenngeweben an den Frucht- und Blattstielen. Blattabzissionswirkung und Defoliation ist bei der Baumwollernte von Bedeutung.

Die Entfaltung der Wirkung der erfindungsgemässen Wirkstoffe erfolgt sowohl über die oberirdischen Pflanzenteile (Kontakwirkung), insbesondere die Blätter, als auch über den Boden, als preemergentes Herbizid (Keimhemmung).

Die Wirkung als starke Wachstumshemmer zeigt sich darin, dass die meisten post-emergent behandelten Pflanzenarten nach dreiwöchiger Versuchsdauer einen Wachstumstillstand zeigen, wobei die behandelten Pflanzenteile eine dunkelgrüne Färbung annehmen. Die Blätter fallen aber nicht ab.

Diese Wuchshemmung tritt bei einigen Pflanzenarten schon bei einer Dosierung von 0,5 kg/ha und darunter auf.

Da nicht alle Pflanzenarten gleich stark gehemmt werden, ist bei Wahl einer bestimmten niederen Dosierung ein selektiver Einsatz möglich.

Die erfindungsgemässen Wirkstoffe sind auch interessante Kombinationspartner für eine Reihe von Herbiziden der Phenylharnstoff- und Triazinreihe in Getreidekulturen, Mais, Zuckerrohr bezw. im Obst- und Weinbau.

In Gebieten mit erhöhter Erosionsgefahr können die erfindungsgemässen Wirkstoffe als Wuchshemmer in den vershiedensten Kulturen eingesetzt werden.

Dabei wird die Unkrautdecke nicht beseitigt, sondern nur so stark gehemmt, dass keine Konkurrenzierung der Kulturpflanzen mehr auftritt.

Die neuen Wirkstoffe der Formel I zeichnen sich überdies durch eine sehr starke pre-emergente Herbizidwirkung aus, sind also auch ausgeprägte Keimungshemmer.

Zum Nachweis der Brauchbarkeit als Herbizide (pre- und post-emergent) und als Wuchshemmer dienten folgende Testmethoden:

*Pre-emergente Herbizid-Wirkung* (Keimhemmung)
Im Gewächshaus wird unmittelbar nach der Einsaat der Versuchspflanzen in Saatschalen die Erdoberfläche mit einer wässerigen Suspension der Wirkstoffe, erhalten aus einem 25%-igen Spritzpulver, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben

2—8 = Zwischenstufen der Schädigung

9 = Pflanzen ungeschädigt (wie unbehandelte Kontrolle).

*Post-emergente Herbizid-Wirkung (Kontakherbizid)*
Eine grössere Anzahl (mindestens 7) Unkräuter und Kulturpflanzen, sowohl monocotyle wie dicotyle, wurden nach dem Auflaufen (im 4-bis-6-Blattstadium) mit einer wässrigen Wirkstoffemulsion in Dosierungen von 0,5; 1; 2 und 4 kg Wirksubstanz pro Hektar auf die Pflanzen gespritzt und diese bei 24°—26°C und 45—60% rel. Luftfeuchtigkeit gehalten. 5 Tage und 15 Tage nach Behandlung wird der Versuch ausgewertet und das Ergebnis wie im pre-emergent-Versuch nach derselben Notenskala bonitiert.

Die geprüften Verbindungen gemäss vorliegender Erfindung zeigten auf einigen Pflanzen ausgeprägte kontaktherbizide Wirkung und auf vielen Pflanzen Wachstumsstillstand als Symptom der wachstumshemmenden Eigenschaften.

*Wuchshemmung bei Gräsern*
In Kunstoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der Formel I bespritzt. Die Wirkstoffmenge betrug umgerechnet 5 kg Aktivsubstanz pro Hektar. 10 und 21 Tage nach Applikation wurde das Wachstum der Gräser beurteilt.

*Wuchshemmung bei Getreide*

In Kunststoffbechern wurde Sommerweizen (Triticum aestivum), Sommergerste (Hordeum vulgare) und Roggen (Secale) in sterilisierter Erde angesät und im Gewächshaus gesogen. Die Getreidesprösslinge werden 5 Tage nach Aussaat mit einer Spritzbrühe des Wirkstoffs behandelt. Die Blattapplikation entsprach 6 kg Wirkstoff pro Hektar. Die Auswertung erfolgt nach 21 Tage.

Die erfindungsgemässen Wirkstoffe bewirken eine merkliche Wuchshemmung sowohl bei den Gräsern wie beim Getreide.

**Patentansprüche**

1. Phenoxy-phenylthio-milchsäurederivate der Formel I

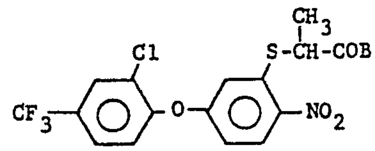

worin

B einen Rest —OR, —SR oder —$NR_1R_2$,

R Wasserstoff, das Kation eines Alkali- oder Erdalkalimetalles oder einer Ammoniumgruppe, einen $C_1$—$C_{18}$ *Alkylrest,* der unsubstituiert oder ein- oder mehrfach durch Halogen, $C_1$—$C_4$ Alkoxy, $C_1$—$C_4$ Alkylthio, $C_3$—$C_8$ Cycloalkyl, Nitro oder Cyano substituiert sein kann, $C_3$—$C_{18}$ *Alkenyl,* unsubstituiert oder durch Halogen substituiert, $C_3$—$C_{18}$ *Alkinyl, Phenyl* oder *Benzyl* unsubstituiert oder substituiert durch Halogen, $C_1$—$C_4$ Alkyl, $C_1$—$C_4$ Alkoxy, $C_1$—$C_4$ Alkylthio, Trifluormethyl, Nitro oder Cyano,

$R_1$ Wasserstoff, *$C_1$—$C_4$ Alkyl,* gegebenenfalls substituiert durch Halogen, Cyano, Hydroxyl oder $C_1$—$C_4$ Alkoxy, *$C_3$—$C_6$ Alkenyl, $C_3$—$C_6$ Alkinyl, $C_1$—$C_4$ Alkoxy,* Phenyl oder Benzyl, gegebenenfalls substituiert durch Halogen, Trifluormethyl, Nitro, Cyano, $C_1$—$C_4$ Alkyl, $C_1$—$C_4$ Alkoxy, $C_1$—$C_4$ Alkylthio,

$R_2$ Wasserstoff, $C_1$—$C_4$ Alkyl oder $C_3$—$C_6$ Alkenyl,

$R_1$ und $R_2$ zusammen mit dem Stickstoffatom an das sie gebunden sind auch einen 5—7 gliedrigen Heterocyclus der auch Sauerstoff, Schwefel oder eine Gruppe $>NR_2$ im ring enthalten kann bedeuten.

2. Als Thiomilchsäurederivat der Formel I, Anspruch 1 $\alpha$ - [3 - (2' - chlor - 4' - trifluormethylphenoxy) - 6 - nitro - phenylthio] - propionsäure.

3. Als Thiomilchsäurederivat der Formel I, $\alpha$ - [3 - (2' - Chlor - 4' - trifluormethylphenyloxy) - 6 - nitro - phenylthio] - propionsäure - methylester.

4. Verfahren zur Herstellung der Phenoxy-phenylthiomilchsäurederivate der Formel I, Anspruch 1, dadurch gekennzeichnet, dass man ein Phenoxy-phenylthio-milchsäurederivat der Formel IV

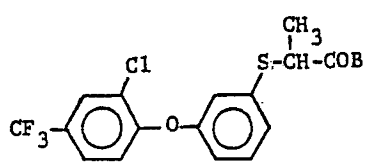

IV

worin B die unter Formel I, Anspruch 1 gegebene Bedeutung hat, bei einer Temperatur, zwischen −10°C und Raumtemperatur mit einem Gemisch aus Salpeter- und Schwefelsäure nitriert.

5. Verfahren zur Herstellung der Phenoxy-phenylthio-milchsäurederivate der Formel I, Anspruch 1, dadurchgekennzeichnet, dass man einen in der 3'-Stellung durch Halogen oder Nitro substituierten 2-Chlor-4-trifluormethyl-(4'-nitro)-diphenyläther der Formel VII,

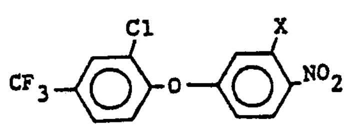

VII

worin X Halogen oder die Nitrogruppe bedeutet, in einem inerten organischen Lösungsmittel in Anwesenheit der äquimolaren Menge einer anorganischen oder organischen Base mit einem Derivat der 2-Thiomilchsäure der Formel VIII,

$$CH_3\text{—}\underset{}{\overset{SH}{\overset{|}{CH}}}\text{—}COB \qquad VIII$$

worin B die unter Formel I gegebene Bedeutung hat, kondensiert.

6. Herbizides Mittel, dadurch gekennzeichnet, dass es als Wirkstoff mindestens ein Phenoxy-phenylthio-milchsäurederivat der Formel I, Anspruch 1 enthält.

7. Die Verwendung der Phenoxy-phenylthio-milchsäurederivat der Formel I, Anspruch 1 oder sie enthaltender Mittel zur Kontrolle von unerwünschtem Pflanzenwachstum.

8. Die Verwendung der Phenoxy-phenylthio-milchsäurederivat der Formel I, Anspruch 1 oder sie enthaltender Mittel zur Regulierung des Pflanzenwachstums.

## Claims

1. A phenoxyphenylthiolactic acid derivative of the formula I

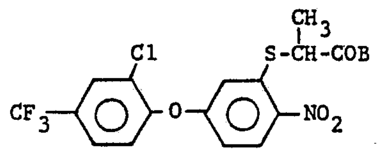

wherein

B is a —OR, —SR or —$NR_1R_2$ radical,

R is hydrogen, the cation of an alkali metal or of an ammonium group, a $C_1$—$C_{18}$alkyl radical which is unsubstituted or substituted by halogen, $C_1$—$C_4$alkoxy, or cyano; $C_3$—$C_4$alkynyl; phenyl or benzyl, unsubstituted or substituted by halogen,

$R_1$ is hydrogen, $C_1$—$C_4$alkyl, $C_3$—$C_4$alkenyl, $C_3$—$C_4$alkynyl, or phenyl or benzyl, unsubstituted or substituted by halogen, and

$R_2$ is hydrogen, $C_1$—$C_4$alkyl or $C_1$—$C_4$alkoxy.

2. α-[3-(2′-Chloro-4-trifluoromethylphenoxy)-6-nitrophenylthio]-propionic acid methyl ester of the formula I.

3. α-[3-(2′-Chloro-4-trifluoromethylphenoxy)-6-nitrophenylthio]-propionic acid methyl ester of the formula I.

4. A process for the production of a phenoxyphenylthiolactic acid derivative of the formula I according to claim 1, which process comprises nitrating a phenoxyphenylthiolactic acid derivative of the formula IV

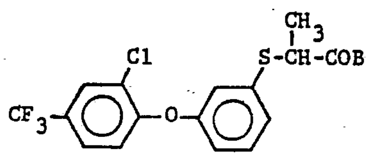

IV

wherein B is as defined for formula I in claim 1, at a temperature between —10°C and room temperature, with a mixture of nitric acid and sulfuric acid.

5. A process for the production of a phenoxyphenylthiolactic acid derivative of the formula I according to claim 1, which process comprises condensing a 2-chloro-4-trifluoromethyl-(4′-nitro)-diphenyl ether, which is substituted in the 3′-position by halogen or nitro, of the formula VII

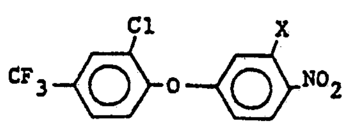

VII

wherein X is halogen or the nitro group, in an inert organic solvent and in the presence of the equimolar amount of an inorganic amount or organic base, with a derivative of 2-thiolactic acid of the formula VIII

SH
|
$CH_3$—CH—COB  VIII

wherein B is as defined for formula I.

6. A herbicidal composition which contains, as active ingredient, at least one phenoxyphenylthiolactic acid derivative of the formula I according to claim 1.

7. A method of controlling undesirable plant growth which comprises applying to plants a phenoxyphenylthiolactic acid derivative of the formula I according to claim 1 or a composition containing such a compound.

8. A method of regulating plant growth which comprises applying to plants a phenoxyphenylthiolactic acid derivative of the formula I according to claim 1 or a composition containing such a compound.

**Revendications**

1. Dérivés phénoxy-phénylthio de l'acide lactique de formule I:

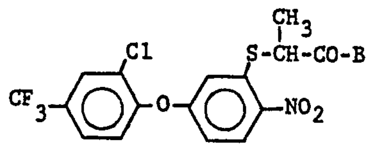

(I)

où B représente un radical —OR, —SR ou —$NR_1R_2$,
R représente un hydrogène; le cation d'un métal alcalin ou d'un groupe ammonium; un radical alcoyle en $C_1$ à $C_{18}$, qui peut être non substitué ou substitué par un halogène, un alcoxy en $C_1$ à $C_4$ ou un cyano; un alcényle en $C_3$ à $C_4$; un alcynyle en $C_3$ à $C_4$; un phényle ou un benzyle non substitué ou substitué par un halogène,
$R_1$ un hydrogène; un alcoyle en $C_1$ à $C_4$; un alcényle en $C_3$ à $C_4$; un alcynyle en $C_3$ à $C_4$; un phényle ou un benzyle, éventuellement substitué par un halogène et
$R_2$ un hydrogène, un alcoyle en $C_1$ à $C_4$ ou un alcoxy en $C_1$ à $C_4$.

2. Comme dérivé thio de l'acide lactique de formule I, l'acide $\alpha$ - (3 - 2' - chloro - 4' - trifluorométhylphénoxy) - 6 - nitrophénylthio) - propionique

3. Comme dérivé thio de l'acide lactique de formule I, l'ester méthylique de l'acide $\alpha$ - (3 - (2' - chloro - 4 - trifluorométhylphénoxy) - 6 - nitrophénylthio) - propionique.

4. Procédé de préparation des dérivés phénoxy-phénylthio de l'acide lactique de formule I de la revendication 1, caractérisé en ce qu'on nitre un dérivé phénoxy-phénylthio de l'acide lactique de formule IV:

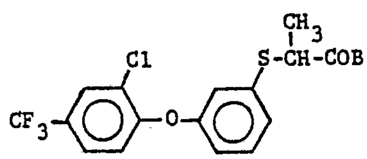

(IV)

où B a la signification donnée dans la formule I de la revendication 1, à une température comprise entre —10°C et la température ambiante, avec un mélange d'acide nitrique et d'acide sulfurique.

5. Procédé de préparation des dérivés phénoxy-phénylthio de l'acide lactique de formule I de la revendication 1, caractérisé en ce qu'on condense un 2-chloro-4-trifluorométhyl-(4'-nitro)-diphényléther de formule VII substitué en position 3' par un halogène ou un nitro

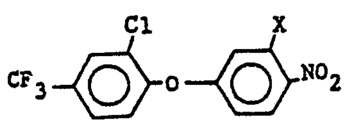

où X représente un halogène ou le groupe nitro, dans un solvant organique inerte en présence d'une quantité équimolaire d'une base inorganique ou organique avec un dérivé de l'acide 2-thiolactique de formule VIII

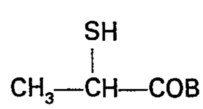

VIII

où B a la signification donnée pour la formule I.

6. Agent herbicide caractérisé en ce qu'il contient comme matière active au moins un dérivé phénoxy-phénylthio de l'acide lactique de formule I de la revendication 1.

7. Application des dérivés phénoxy-phénylthio de l'acide lactique de formule I de la revendication 1 ou des agents qui les contiennent pour maîtriser la croissance végétale indésirable.

8. Application des dérivés phénoxy-phénylthio de l'acide lactique de formule I de la revendication 1 ou des agents qui les contiennent à la régulation de la croissance des plantes.